# FASCICULE DE BREVET EUROPEEN

(11) **EP 0 653 196 B1**
(45) Date de publication et mention de la délivrance du brevet: **06.05.1998**
(21) Numéro de dépôt: 94402501.4
(22) Date de dépôt: 07.11.1994
(51) Int. Cl.: A61F 5/441

(54) **Dispositif de dégazage et de contrôle de la pression interne d'une poche de recueil pour stomie**
Ventil und innendrucksteuervorrichtung für Ostomiebeutel
Venting and internal pressure control device for ostomy pouch

(30) Priorité: 08.11.1993 FR 9313246; 11.05.1994 FR 9405805
(43) Date de publication de la demande: 17.05.1995
(73) Titulaire: B. BRAUN BIOTROL, 92107 Boulogne Billancourt (FR)
(72) Inventeur: Holtermann, Henri, F-64500 Saint-Jean-de-Luz (FR); Hamelin, Claude, F-64310 Ascain (FR)
(74) Mandataire: Orès, Bernard

(56) Documents cités:
- DE-A- 3 304 311
- FR-A- 2 514 636
- GB-A- 2 094 153
- GB-A- 2 116 433
- US-A- 4 451 258

## Description

L'Invention concerne un dispositif de dégazage et de contrôle de la pression interne d'une poche de recueil pour stomie et une poche munie d'un tel dispositif.

On connaît déjà dans de nombreuses réalisations, voir, par exemple, GB-1-416 594 ou US-3 865 109, des poches de recueil pour stomie munies de dispositifs de dégazage qui permettent à la fois l'évacuation des gaz intestinaux ayant pénétré dans la poche et le maintien dans cette dernière d'une valeur de pression résiduelle qui s'oppose à ce que les feuilles ou films constitutifs de la poche ne se collent l'un à l'autre, empêchant ainsi un recueil satisfaisant des excrétions solides ou liquides. C'est le cas en particulier du dispositif selon la Demande de Brevet FR 2 514 636 qui comporte un corps de soupape pourvu de deux orifices se faisant face, l'un communiquant avec l'intérieur de la poche et entouré d'un siège de soupape, et l'autre communiquant avec l'extérieur. A l'intérieur de ce corps , entre les deux orifices se trouve un élément obturateur de soupape ayant la forme d'un disque pourvu, sur son pourtour, d'un certain nombre de languettes élastiques qui s'appuient contre la paroi intérieur du corps de soupape et pressent l'élément obturateur contre le siège d'évent.

On connaît également, par exemple par EP-B1-0 294 257 ou EP-A-0 116 363, des poches de recueil qui, outre des dispositifs de dégazage, sont également munies de filtres placés à l'intérieur ou à l'extérieur de la poche et dont l'épaisseur est maintenue à une valeur aussi faible que possible pour qu'ils puissent remplir leur rôle tout en n'ayant que peu d'incidence sur la saillie que forme la poche sur le corps et sous les vêtements d'un utilisateur. Dans la mesure, toutefois, où les dispositifs connus, en particulier par les documents cités en dernier lieu, sont intégrés à la poche qu'ils équipent, ils ne peuvent pas être utilisés sans filtre, ni être rendus opératoires soit automatiquement, - c'est-à-dire sans intervention du patient qui porte la poche -, soit à la volonté de celui-ci. Ils ne présentent pas, de ce fait, les caractéristiques de souplesse d'utilisation qui sont requises pour appareiller des patients en fonction de leurs desiderata ou de ceux du personnel médical ou pour pouvoir prendre en compte des conditions très différentes d'utilisation de la poche de recueil.

GB-A-2 094 153 décrit un dispositif de dégasage comportant un boîtier, un clapet relié au boîtier et un couvercle adapté à s'appliquer sur le boîtier pour fixer le dispositif à la poche de stomie. Le couvercle n'est pas fait pour être enlevé pendant l'usage.

Le problème se pose, par conséquent, de fournir un dispositif de dégazage et de contrôle de la pression interne d'une poche de recueil pour stomie propre à pallier les inconvénients qui viennent d'être rappelés.

C'est, d'une façon générale, un but de l'Invention de fournir un dispositif de dégazage et de contrôle de la pression interne d'une poche de recueil pour stomie qui permette de résoudre le problème posé.

C'est, encore, un but de l'Invention, de fournir un tel dispositif dont l'utilisation soit simple et, de ce fait, puisse être utilisé par tous les patients ayant subi une intervention chirurgicale comme une colostomie ou une iléostomie.

C'est, également, un but de l'Invention de fournir un tel dispositif exempt de tout risque de mauvais fonctionnement, lequel peut entraîner des situations très embarrassantes pour des patients ayant subi une intervention chirurgicale du type de celles mentionnées ci-avant.

C'est, toujours, un but de l'Invention de fournir un tel dispositif dont les contraintes de fabrication, notamment de réalisation des pièces et d'assemblage, sont en rapport avec une exploitation industrielle.

C'est, enfin, un but de l'Invention de fournir une poche de recueil pour stomie à laquelle un dispositif tel que défini ci-dessus peut être aisément associé.

Ce problème est résolu par un dispositif de dégazage, notamment pour poche de stomie, constitué par un boîtier essentiellement plat comportant un fond ayant une face interne au boîtier et une face externe au boîtier, adapté à être fixé par ladite face externe sur une face exposée de ladite poche et muni d'une ouverture communiquant avec ladite poche, ainsi qu'un clapet du type fermé au repos, ledit clapet étant formé par un organe de fermeture adapté à s'appliquer sur ladite ouverture dudit fond du côté de ladite face interne et d'un seul tenant avec des moyens élastiques pour repousser ledit organe contre ledit fond avec une force de rappel prédéterminée, caractérisé en ce que le boîtier comporte en outre un couvercle amovible adapté à fermer ledit fond et en ce que le clapet est solidaire dudit couvercle.

De la sorte en effet, la fabrication du présent dispositif se trouve facilitée par rapport à celui enseigné par la Demande FR 2 514 636 citée ci-avant.

En effet, il ne met pas en jeu le clapet indépendant à insérer dans un corps de soupape alors que sa petite taille le rend peu maniable. L'assemblage peut dès lors être effectué à l'usine par exemple en emboîtant simplement le couvercle sur le fond du boîtier. Il peut aussi être effectué par l'utilisateur lui-même. La simplification ou l'absence d'assemblage à l'usine aboutit à un gain sur les coûts de production d'autant plus notable que le nombre de dispositifs fabriqués est important.

Avantageusement, les moyens élastiques consistent en au moins un méplat élastiquement déformable s'étendant entre ledit couvercle et ledit organe de fermeture. Un tel méplat s'obtient aisément en fabriquant le fond et le couvercle sous forme d'une ébauche d'un seul tenant, de préférence par moulage par injection d'une matière plastique, des ponts de matière reliant dans l'ébauche ledit fond et ledit couvercle de sorte que la distance entre ledit couvercle et ledit organe de fermeture est supérieure à la distance entre ledit fond et ledit couvercle dans le boîtier définitif obtenu en rompant lesdits ponts de matière et en fermant ledit fond avec ledit couvercle.

A la demande, un filtre comportant un trou central est logé dans ledit boîtier de sorte qu'il est pincé entre ledit clapet et ledit fond, son trou venant dans le prolongement de ladite ouverture dudit fond. Le cas échéant, l'utilisateur lui-même place le filtre dans l'évent. Il peut le faire alors qu'il porte la poche. Il s'ensuit une grande flexibilité d'emploi.

Avantageusement, l'ensemble formé par ledit couvercle et ledit organe de fermeture est conformé pour permettre l'actionnement manuel dudit clapet. Cela est avantageusement obtenu par un dispositif caractérisé en ce que ledit boîtier est cylindrique et en ce que ledit couvercle est assujetti audit fond de façon à pouvoir être déplacé relativement audit fond grâce à des moyens d'actionnement manuel selon un mouvement de rotation autour de l'axe (A) du boîtier.

De préférence alors, le couvercle est assujetti sur le fond par emboîtement et les moyens d'actionnement manuel consistent en une oreille en saillie radiale sur le couvercle.

Il peut alors être utile de prévoir une autre oreille saillant radialement sur ledit fond et propre à servir de repère d'indexation pour celle du couvercle. Cette structure est particulièrement adaptée à la nécessité que le dispositif soit plat afin d'être discret sous les vêtements. Il est en effet alors facile de pousser l'oreille du couvercle pour le faire tourner, et cela à tout moment tandis que l'utilisateur porte la poche.

Selon un premier mode de réalisation, ledit organe de fermeture est solidaire d'au moins un doigt radial adapté à circuler, lorsque lesdits moyens d'actionnement manuel sont actionnés, dans un guide dudit fond s'étendant circonférentiellement et de forme telle que ledit doigt peut, selon sa position dans ledit guide, être libre ou être soulevé à l'encontre desdits moyens élastiques le long d'une rampe du guide.

Avantageusement, il existe aussi une position dans laquelle ledit doigt peut être verrouillé sous un crochet dudit guide.

Il s'ensuit que la rotation du couvercle par rapport au fond que provoque l'utilisateur, en amenant le doigt à se déplacer le long de la rampe, résulte en un déplacement axial de l'organe de fermeture qui assure l'ouverture du clapet d'évent. Cette disposition est également particulièrement bien adaptée à l'exigence de discrétion qui, en imposant une faible épaisseur globale au dispositif, veut que le déplacement axial à provoquer soit réduit. La structure retenue ici autorise ce faible déplacement même si la rotation du couvercle est plus substantielle et ainsi plus commode à réaliser.

Selon un second mode de réalisation, ledit organe de fermeture consiste en un pointeau, le couvercle comportant également au moins un bossage tandis que ledit fond comporte au moins une rampe hélicoïdale sur laquelle ledit bossage est adapté à circuler entre une position basse et une position haute lorsque le couvercle est tourné par rapport audit fond de sorte que, dans la position basse, ledit pointeau est rappelé par les moyens élastiques pour fermer ladite ouverture tandis que, dans la position haute, ledit pointeau est repoussé hors de l'ouverture à l'encontre desdits moyens élastiques.

Avantageusement, il y a trois bossages et trois rampes disposées selon des directions radiales faisant 120° les unes par rapport aux autres.

Avantageusement encore, lesdits bossages sont solidaires d'un plot qui porte ledit pointeau à son extrémité, les extrémités libres desdits bossages étant en retrait par rapport audit pointeau de façon que ledit pointeau pénètre dans ladite ouverture lorsque lesdits bossages sont en position basse.

De même que, dans le premier mode de réalisation, la rotation du couvercle résulte en un déplacement axial du pointeau qui aboutit à l'ouverture du clapet.

Dans ce second mode de réalisation, ledit boîtier comporte de préférence un espace intérieur adapté à recevoir un filtre et limité par une paroi cylindrique comportant des évidements axiaux communiquant avec des fenêtres du dessus du couvercle de sorte que les gaz peuvent s'échapper malgré la présence d'un filtre à l'intérieur du boîtier. Il est intéressant que le fond présente alors un relief intérieur adapté à pincer le filtre de sorte que les gaz sont forcés de passer au travers du filtre pour s'échapper par les évidements et les fenêtres.

Avantageusement, le couvercle est assujetti sur le fond par un emboîtement à baïonnette. Pour cela, ledit fond et ledit couvercle présentent de préférence des rebords adaptés à collaborer de sorte qu'une fois ledit fond et ledit couvercle emboîtés, les rebords coopèrent pour les empêcher de se séparer, lesdits rebords s'étendant sur un secteur d'environ 90° et étant disposés de façon diamétralement opposée sur le couvercle et sur le fond. Alors, le couvercle peut s'insérer à l'intérieur du fond, les rebords du couvercle saillant vers l'extérieur tandis que les rebords du fond saillent vers l'intérieur. Au moins un rebord se prolonge de préférence en une colonne axiale de butée adaptée à empêcher que fond et couvercle ne puissent tourner au-delà d'une certaine valeur et que les bossages ne quittent par suite ladite rampe.

L'Invention se rapporte aussi à une poche de recueil comportant un dispositif de dégazage et de contrôle de la pression interne comme décrit précédemment.

L'Invention sera bien comprise par la description qui suit, faite à titre d'exemple et en référence au dessin annexé, dans lequel :
- la Figure 1 est une vue schématique, en coupe, d'un premier mode de réalisation du dispositif selon l'Invention associé à une poche de recueil ;
- la Figure 2 est une vue en perspective éclatée du dispositif de la Figure 1 dont un couvercle est montré en vue partiellement écorchée ;
- la Figure 3 est une vue de dessus, avec arrachement partiel, du dispositif des Figures précédentes ;
- la Figure 4 est une vue analogue à celle de la Figure 2 mais pour une autre condition ;
- la Figure 5 est une vue en coupe diamétrale du dispositif des Figures précédentes utilisé seul ;
- la Figure 6 est une vue en coupe selon la ligne 6-6 de la Figure 3 pour le dispositif des Figures précédentes utilisé avec un filtre ;
- la Figure 7 est une vue en coupe selon la ligne brisée 7-7 de la Figure 4 ;
- la Figure 8 est une vue partielle de dessus d'une variante du dispositif des Figures précédentes ;
- la Figure 9 montre selon une vue en perspective un second mode de réalisation du dispositif de dégazage selon l'Invention qui est conformé en un boîtier constitué d'un fond et d'un couvercle ;
- la Figure 10 montre, toujours selon une vue en perspective, le couvercle du dispositif de la Figure 1. Par rapport à la Figure 1, le couvercle est toutefois montré retourné ;
- la Figure 11 montre, encore selon une vue en perspective, le fond du dispositif de la Figure 1 ;
- la Figure 12 montre une vue de dessus du dispositif précédent en position ouverte du clapet. Son couvercle est en partie déchiré pour qu'une partie du fond soit visible ;
- la Figure 13 montre une coupe selon le plan I-I de la Figure 12 du dispositif précédent dans la même position ouverte du clapet ;
- la Figure 14 montre une autre coupe selon, cette fois, le plan II-II de la Figure 12 du dispositif précédent toujours en position ouverte du clapet ;
- la Figure 15 montre une vue de dessus du dispositif précédent en position fermée du clapet ;
- la Figure 16 montre une coupe selon le plan I-I de la Figure 7 du dispositif précédent dans la même position fermée du clapet.

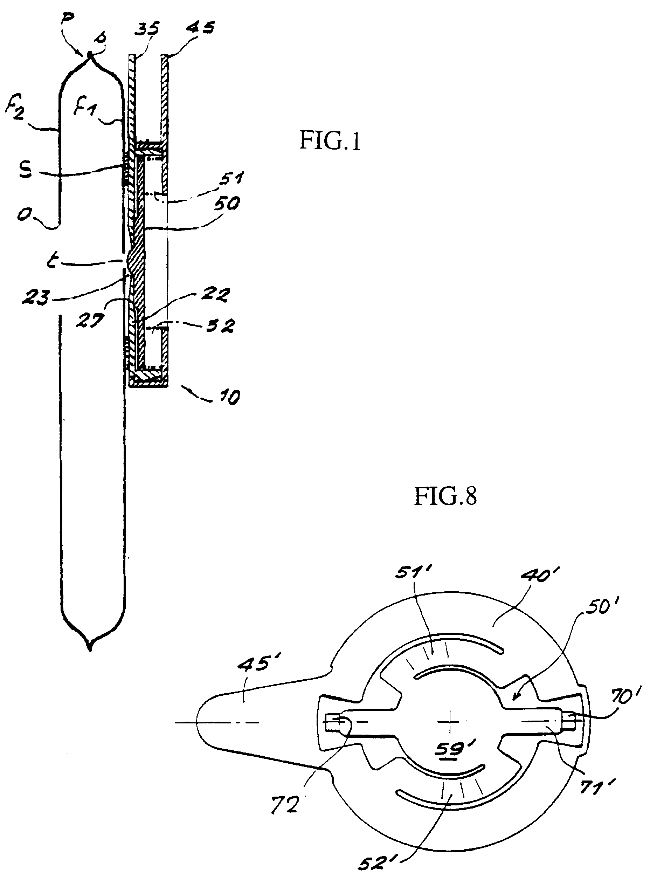

Un premier mode de réalisation du dispositif selon l'Invention, 10, de dégazage et de contrôle de la pression interne d'une poche de recueil pour stomie P est montré sur les Figures 1 à 7. Il est destiné à être fixé sur la face exposée de la poche laquelle, du type jetable ou vidangeable, est généralement constituée par deux feuilles ou films de matière plastique imperméables aux odeurs, f₁, f₂, soudés entre eux sur leur périphérie s. De façon en soi connue, un des films f₁, f₂ comporte une ouverture O, propre à être mise en communication avec la stomie lorsque la poche est assujettie au corps du patient, soit par un système de raccord, soit à l'aide d'un patin adhésif, soit par une ceinture ou, encore, par une combinaison de ces divers moyens. La poche P, qui recueille les excrétions solides ou liquides, recueille également les gaz intestinaux qui doivent être évacués pour éviter que la poche n'augmente exagérément de volume. Une certaine pression résiduelle doit cependant être maintenue dans la poche pour pallier l'inconvénient qui résulterait du rapprochement et du collage éventuel l'un à l'autre des deux films f₁, f₂ et qui s'opposerait à l'entrée dans la poche, par l'ouverture O, des excrétions solides ou liquides à recueillir.

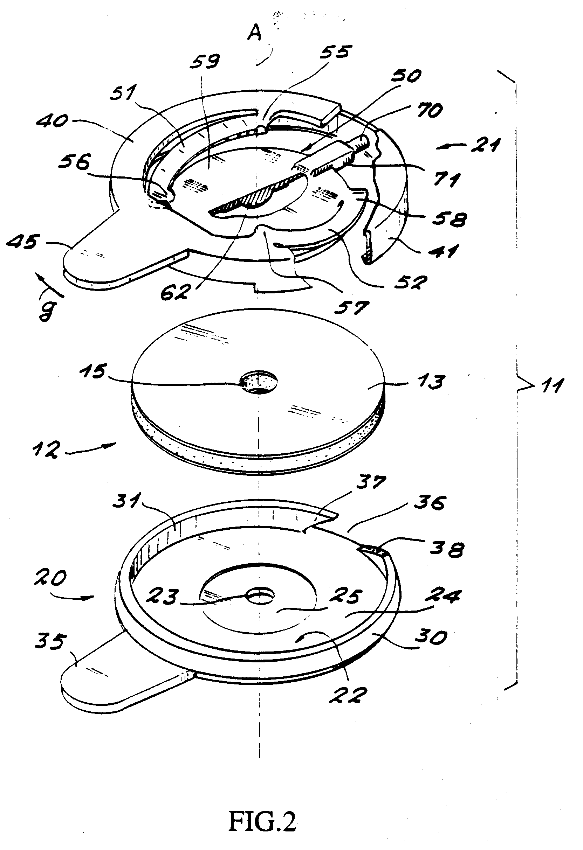

Pour ce faire, le dispositif selon l'Invention, 10, comprend un boîtier essentiellement plat 11 (épaisseur typique 5 mm), Figure 2, dans lequel peut être logé un filtre 12 absorbant les odeurs et réalisé à partir d'un substrat poreux, comme de la mousse, un matériau tissé ou non tissé, imprégné de charbon actif ou d'un autre désodorisant et qui est conformé suivant un disque de faible épaisseur recouvert sur chacune de ses faces d'un film imperméable au gaz, 13 et 14, respectivement, l'ensemble présentant en son centre un trou 15.

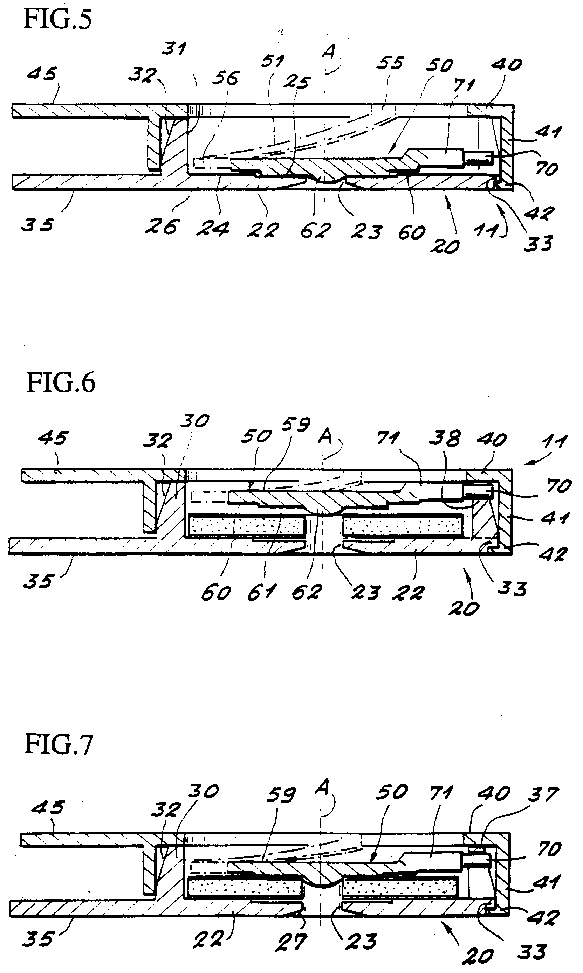

Le boîtier 11 résulte de l'assemblage entre eux d'un fond 20 et d'un couvercle 21 avantageusement réalisés par moulage, de préférence par injection, d'une matière plastique élastiquement déformable comme du polyéthylène, du polyamide, un ABS, ... etc, le fond et le couvercle étant reliés entre eux par un fin cordon de matière aussi longtemps qu'ils ne sont pas appariés. Le boîtier 11 a une forme générale cylindrique d'axe A (d'un diamètre de l'ordre de 30 mm) et les surfaces de ses éléments qui se trouvent à l'intérieur du boîtier seront dites par la suite internes, tandis que les autres seront dites externes, par opposition. Le fond 20, qui est destiné à être fixé sur la face exposée de la poche, par exemple par une soudure annulaire S ou par collage, comporte une partie plane 22, à contour circulaire, percée en son centre d'une ouverture 23 laquelle est entourée, sur la face interne 24 du fond par un lamage circulaire 25 et, sur la face externe 26, par un lamage tronconique 27, Figure 7. De la partie 22 est solidaire une paroi 30, à surface interne 31 cylindrique, d'axe A et à surface externe 32 tronconique (Figures 5 à 7) pour ménager un rebord 33 d'accrochage par emboîtement du boîtier 21. Une oreille radiale 35 fait saillie dans le plan de la partie 22, à l'extérieur de celle-ci, tandis que la paroi 30 présente un évidement ou guide 36, diamétralement opposé à l'oreille 35, et qui ménage à une de ses extrémités un bec ou un crochet 37 et, à son autre extrémité, une rampe inclinée 38.

Le couvercle 21, de même contour circulaire que celui du fond 20, comprend une marge ou bordure annulaire 40, en partie interrompue, de même axe A que celui du fond 20 lorsque les deux parties sont réunies entre elles par accrochage sur le rebord 33 de l'extrémité 42 d'une paroi cylindrique 41 (Figures 5 à 7) ; l'accrochage est tel que le couvercle 21 peut tourner par rapport au fond 20 autour de l'axe A, par manoeuvre d'une oreille radiale 45 s'étendant dans le plan de la bordure 40, à l'opposé de sa partie interrompue et à l'extérieur de ladite bordure, les oreilles 45 et 35 étant sensiblement parallèles lorsque le dispositif est assemblé, Figures 5 à 7.

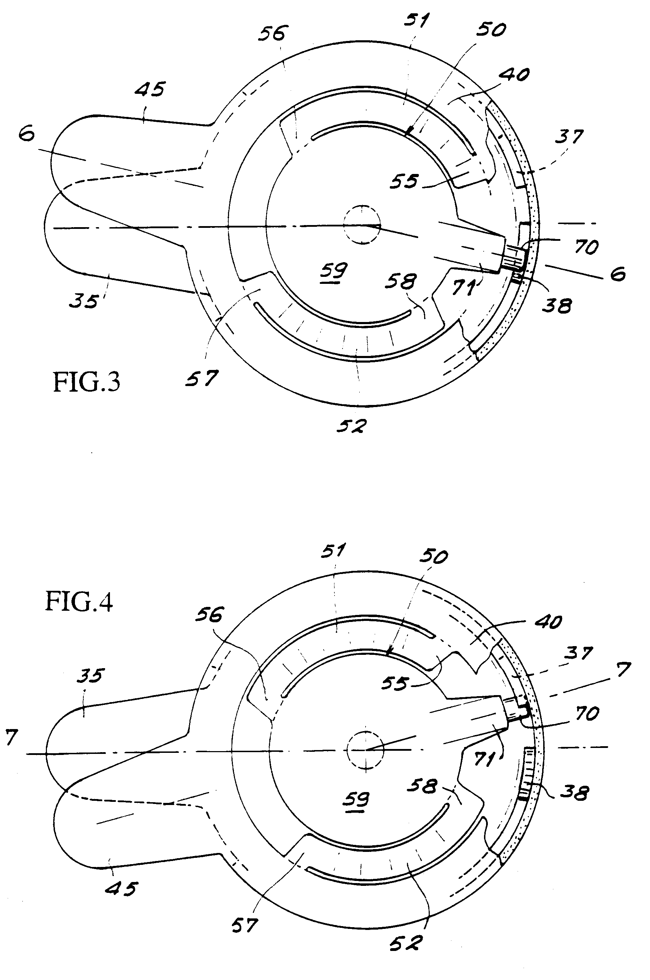

A l'intérieur de l'espace limité par la paroi cylindrique 41 et la marge 40, le couvercle 21 comporte un clapet 50 formé par un organe de fermeture solidaire de deux bandes élastiquement déformables 51 et 52, Figures 3 et 4, chacune quelque peu en arc d'hélice et qui s'étendent :
. pour la bande 51, entre une extrémité 55 au niveau de la marge 40 et une extrémité 56 au niveau du clapet 50 ; et
. pour la bande 52, entre une extrémité 57 solidaire de la marge 40 et une extrémité 58 solidaire du clapet 50.

Ce dernier, dont la position dans la condition de repos est comme montré sur la Figure 5, c'est-à-dire au voisinage de l'extrémité libre 42 de la paroi 41, présente un contour généralement circulaire, avec une face 59 en regard de la marge 40 plane, tandis que la face opposée, 60, est conformée suivant un ressaut circulaire 61, de forme complémentaire à celle du lamage 25 du fond 20 ; au centre du ressaut 61 une saillie 62, sous forme d'un plot semi-sphérique, est propre à obturer l'ouverture 23 du fond 20 lorsque, comme montré sur la Figure 5, ledit ressaut 61 est logé dans le lamage 25. Le clapet 50 constitue ainsi un clapet du type fermé au repos sollicité par des moyens élastiques qui le repoussent en direction de la face interne du fond avec une force de rappel prédéterminée exercée par les deux bandes déformables 51 et 52.

Ladite force de rappel peut être obtenue directement, lors de l'assemblage du fond 20 et du couvercle 21, si ledit fond et ledit couvercle sont fabriqués sous forme d'une ébauche d'un seul tenant avec des ponts de matière reliant dans l'ébauche le fond et le couvercle de manière telle que la distance entre ledit couvercle 21 et le clapet 50 soit supérieure à la distance entre le fond 20 et le couvercle 21 dans le boîtier 11, définitif, obtenu en rompant lesdits ponts de matière lors de l'assemblage du fond et du couvercle, les bandes élastiquement déformables 51 et 52 tendant à toujours reprendre la forme qu'elles avaient dans l'ébauche et qui représente pour elles l'état de repos.

Le clapet 50 comporte également, sensiblement dans le prolongement de la direction moyenne de l'oreille 45, un doigt cylindrique 70 qui forme l'extrémité distale d'une partie 71 dont la direction est ainsi radiale et qui est logé en regard de la partie interrompue de la marge 40.

Conformément à l'Invention, le dispositif 10 qui vient d'être décrit peut être utilisé sans le filtre 12, - et il est alors dans la condition montrée sur les Figures 1 ou 5 en position de repos -, avec son ouverture 23 au droit d'un trou t pratiqué dans le film f₁ de la poche P. Le dispositif peut aussi, et à la discrétion du patient, être utilisé avec un filtre 12 lequel est placé, préalablement à leur assemblage, entre le fond 20 et le couvercle 21 avec le trou 15 du filtre en regard du trou 23 du fond 20.

Le fonctionnement d'un dispositif selon l'Invention est le suivant :

### Sans filtre.

Le boîtier 11, fermé, avec les oreilles 35 et 45 en regard l'une de l'autre est fixé sur la poche P, le clapet 50 étant dans la condition montrée sur la figure 5 c'est-à-dire celle en laquelle le ressaut circulaire 61 est placé dans le lamage 25 et en laquelle le plot semi-sphérique 62 obture l'ouverture 23. Dans cette condition, le doigt 70 est dans l'évidement 36 et, lorsque la pression des gaz intestinaux dans la poche P devient supérieure à la force de rappel prédéterminée que développent les bandes 51 et 52, le clapet se soulève, pour le dégazage, puis reprend ensuite sa position de fermeture avant que la pression interne de la poche n'ait chuté à la pression atmosphérique, sans intervention du patient : en demeurant à une valeur équilibrant la force de rappel des bandes 51 et 52, la pression interne garantit que les parois de la poche ne se collent pas l'une à l'autre. Le fonctionnement est automatique.

Lorsque l'oreille 45 est mise en rotation pour amener le doigt 70 sous le bec 37, le clapet 50 est bloqué en position de fermeture et tout dégazage est interdit.

Lorsque, par contre, une rotation en sens inverse de celle qui vient d'être décrite, c'est-à-dire une rotation dans le sens de la flèche g de la Figure 2, amène le doigt 70 sur la rampe inclinée 38, la coopération du doigt 70 et de cette rampe provoque le mouvement ascendant du clapet 50 et, par suite, l'échappement des gaz recueillis dans la poche P à l'intérieur de laquelle la pression diminue : la dépressurisation est provoquée à la volonté de l'utilisateur.

### Avec filtre.

Lorsque le filtre 12 est mis en place dans le boîtier 11, préalablement à la fermeture de celui-ci, -soit lors de l'assemblage du dispositif en usine, soit alors que la poche est en place sur un patient-, il est placé entre le fond 20 et le clapet 50, la condition de repos étant celle montrée sur la Figure 7 en laquelle le plot semi-sphérique 62 coopère avec le trou 15 du filtre, pour fermer ce trou, alors que l'ouverture 23 en regard du trou 15 permet le passage des gaz recueillis par la poche dans le filtre.

Dans la condition en laquelle les oreilles 35 et 45 sont en regard l'une de l'autre, le dégazage de la poche P s'effectue au travers du filtre 12, aussi longtemps que la pression reste à une valeur normale, le pincement du filtre 12 garantissant une bonne étanchéité du clapet lorsque celui-ci est fermé, le cheminement des gaz entre le fond 20 et le filtre étant entravé par le coude formé par le lamage 25. L'apparition d'une surpression provoque le déplacement du clapet 50, indépendamment de toute action du patient puis, après dégazage et abaissement de la pression interne de la poche à une valeur équilibrant la force de rappel prédéterminée des bandes élastiques 51 et 52, lesdites bandes ramènent le clapet 50 sur le filtre.

Lorsque l'oreille 45 est mise en rotation dans le sens inverse de celui montré par la flèche g, et que le doigt 70 est engagé sous le bec 37, les gaz recueillis dans la poche P passent au travers du filtre 12, toute fuite massive de gaz étant interdite aussi longtemps qu'elle n'est pas provoquée par le patient.

De façon analogue à celle décrite ci-dessus pour le fonctionnement sans filtre, une mise en rotation du couvercle 21, à l'aide de l'oreille 45 actionnée dans le sens de la flèche g, provoque un mouvement ascendant du clapet 50, en raison de la coopération du doigt 70 et de la rampe 38 qui provoque la dépressurisation, à la volonté du patient, et avec passage principal des gaz recueillis par l'ouverture 23 du filtre 12.

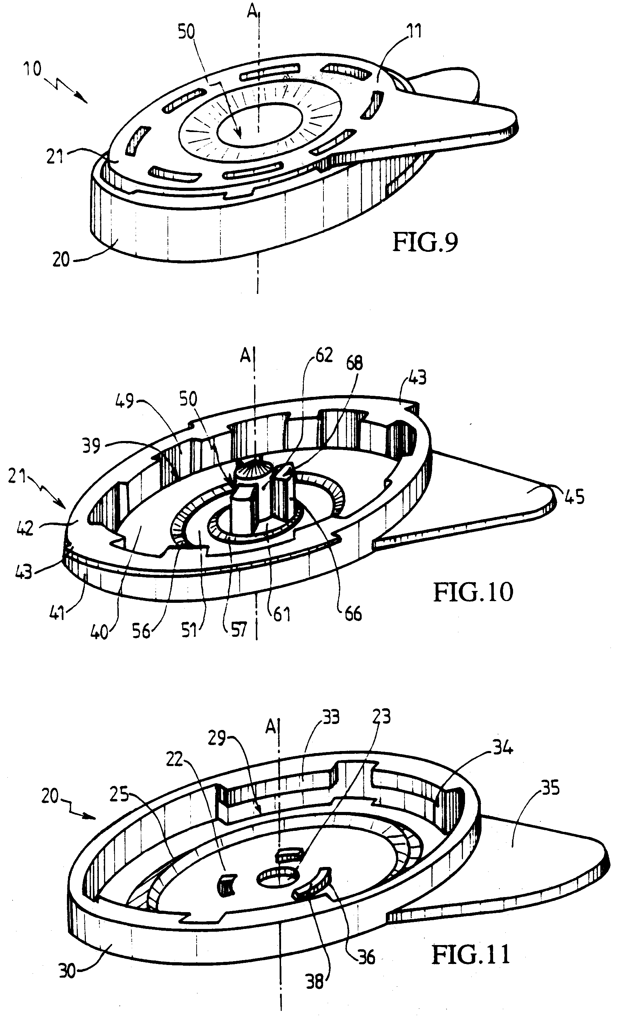

Dans la variante illustrée schématiquement sur la Figure 8 où les parties correspondantes portent les mêmes références que celles de la réalisation précédente mais frappées par l'indice " ' ", le clapet 50' solidaire de deux bandes élastiquement déformables 51' et 52' comporte deux doigts 70' et 72, diamétralement opposés, propres à coopérer, chacun, avec un évidement ou guide correspondant du fond du dispositif. Le fonctionnement d'un tel mode d'exécution à structure bien équilibrée est le même que celui de la réalisation décrite ci-dessus avec ou sans filtre.

Le dispositif selon l'Invention présente ainsi une grande souplesse d'utilisation permettant de satisfaire aux desiderata des utilisateurs ou de ceux du personnel médical pour prendre en compte des conditions très différentes d'utilisation de la poche de recueil.

Un second mode de réalisation du dispositif 10 selon l'Invention, de dégazage et de contrôle de la pression interne d'une poche de recueil pour stomie est montré sur les Figures 9 à 16. Par rapport au premier mode de réalisation, les éléments analogues ont reçu les mêmes numéros de référence.

La Figure 9 montre une vue en perspective du boîtier 11 constituant un mode de réalisation de ce dispositif 10. Ce boîtier 11 est cylindrique avec un axe de révolution A. Il est réalisé de préférence en matière plastique et est constitué d'un fond 20 et d'un couvercle 21 qui s'adaptent avantageusement l'un à l'autre par emboîtement. Comme cela sera mieux exposé ci-dessous, le fond 20 comporte une ouverture 23 (cf. Figure 11) tandis que le couvercle comporte un clapet 50 adapté à coopérer avec cette ouverture 23 pour l'ouvrir ou la fermer selon que la pression des gaz dans la poche augmente ou diminue, et cela principalement lorsque l'utilisateur le désire.

Ce couvercle 21 est mieux visible sur la Figure 10 qui, au demeurant, le montre retourné de sorte que l'intérieur du couvercle soit visible. C'est ainsi qu'apparaît une paroi cylindrique 41 ayant une extrémité libre 42. Extérieurement et à l'opposé de cette extrémité, une oreille 45 saille radialement sur la paroi 41. L'extrémité 42 de la paroi 41 est par ailleurs munie de deux rebords sectoriels 43 saillant radialement vers l'extérieur. Ils s'étendent chacun sur un secteur d'environ 90° et sont diamétralement opposés. Intérieurement, des évidements axiaux 49 - en l'occurrence huit - sont pratiqués dans la paroi 41. La paroi 41 entoure une partie plane ou marge 40 du couvercle 21 ayant la forme d'une couronne périphérique. Des fenêtres 39 percées dans la marge 40 viennent ainsi dans le prolongement des évidements axiaux 49.

Au centre du couvercle 21, se trouve le clapet 50 qui est constitué tout d'abord d'une couronne 51 de matière plus mince que celle de la marge 40. Elle est bordée extérieurement par un décrochement 56 assurant la transition entre la marge 40 et la couronne amincie 51. De même, la couronne amincie 51 est bordée intérieurement par un autre décrochement 57. Ainsi la couronne 51 permet-elle à un disque 61 au centre du clapet de se déplacer par rapport à la marge 40. L'ensemble marge 40 - couronne amincie 51 - disque 61 est moulé de telle sorte qu'en position de repos, le disque 61 se trouve, par rapport au plan de la marge 40, dans un plan situé plus à l'intérieur du couvercle 21. Dès lors, la couronne amincie 51 joue le rôle de moyens élastiques qui assurent le rappel du disque 61 vers l'intérieur du boîtier 11.

L'organe de fermeture du clapet 50 est par ailleurs constitué par le disque 61 sur lequel saille un plot 62 entouré de trois bossages latéraux 66 s'étendant dans des directions faisant 120° les unes par rapport aux autres. Le plot 62 a une extrémité libre terminée par un pointeau dont la taille est choisie pour pénétrer en partie dans l'ouverture 23 et ainsi la fermer. Ce dernier dépasse par rapport aux extrémités libres 68 des bossages 66 qui sont, de leur côté, planes.

Le fond 20 est montré sur la Figure 11. Il comporte une paroi cylindrique 30. Extérieurement, une oreille 35 s'étend radialement à un niveau opposé à l'extrémité libre de la paroi 30. Intérieurement, et cette fois de niveau avec l'extrémité libre de la paroi 30, des rebords sectoriels 33 saillent radialement vers l'intérieur. Chacun d'eux s'étend sur un secteur d'environ 35°. Il y a deux couples de rebords 33 s'étendant chacun sur un secteur total d'environ 90° qui sont diamétralement opposés. Un rebord 33 de chaque couple se prolonge par une colonne axiale 34 faisant corps avec la paroi 30.

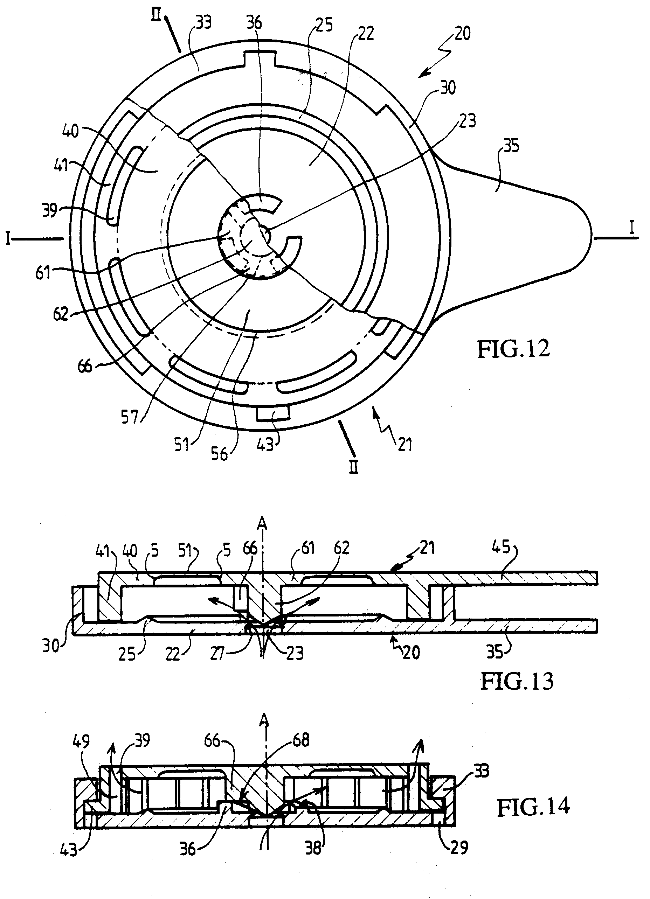

La paroi 30 du fond 20 entoure une partie plane 22 qui comporte des jours 29 disposés en regard des rebords 33. Ils sont à la vérité rendus nécessaires pour le moulage du fond 20 par injection. Au centre de la partie plane 22 se trouve l'ouverture 23 destinée à communiquer avec l'intérieur de la poche pour stomisés. Comme cela est mieux visible sur les coupes notamment des Figures 13 et 14, l'ouverture 23 est munie sur son rebord intérieur au boîtier d'une lèvre d'étanchéité 27. Un relief annulaire 25 saille sur la partie plane 22 à peu près à mi-chemin entre la paroi cylindrique 30 et l'ouverture 23. Enfin trois guides 36 en forme de rampes hélicoïdales 38 s'élevant par rapport à la partie plane 22 du fond 20 par exemple, comme représenté, dans le sens inverse des aiguilles d'une montre sont disposés autour de l'ouverture 23. Ils sont destinés à collaborer avec les bossages 66 du clapet 50 lorsque fond 20 et couvercle 21 sont emboîtés pour former le boîtier 11. Aussi leur distance radiale par rapport à l'axe A est-elle égale à celle des bossages 66 et comme eux dans ce mode de réalisation, ils sont disposés dans des directions faisant 120° les unes par rapport aux autres.

Les Figures suivantes permettent de mieux se rendre compte de la façon dont couvercle 21 et fond 20 sont emboîtés. La paroi 41 du couvercle 21 prend place à l'intérieur de la paroi 30 du fond 20. Lors de l'emboîtage proprement dit, il est nécessaire de présenter les rebords sectoriels 43 du couvercle 21 dans l'espace libre entre les rebords sectoriels 33 du fond 20. Puis, en faisant tourner le couvercle 21 par rapport au fond 20, on fait passer les rebords 43 du couvercle 21 sous les rebords 33 du fond 20 si bien que couvercle 21 et fond 20 deviennent solidaires selon un emboîtage à baïonnette. Pour faire tourner le couvercle 21 par rapport au fond 20, il est possible d'utiliser les oreilles radiales 35 et 45 qui servent donc de moyens d'actionnement.

Une fois, l'emboîtement réalisé, le boîtier délimite intérieurement un espace 19 qui est à même d'accueillir un filtre. Bien que celui-ci ne soit pas représenté sur les Figures, il est clair qu'une fois inséré dans le boîtier 11, il est pincé entre le couvercle 51 et le fond 20 essentiellement au niveau du relief 25 qui sert à cette fin.

Les Figures 12 à 14 se rapportent à la position d'ouverture du clapet 50. Notamment sur la vue de dessus de la Figure 12 où les bossages 66 et les rampes 36 sont représentés en pointillés, on voit qu'elle est réalisée lorsque couvercle 21 et fond 20 ont été tournés l'un par rapport à l'autre de sorte que l'extrémité 68 des bossages 66 repose sur la partie la plus élevée des rampes 38, c'est-à-dire est en position haute. Grâce à la mobilité offerte par la couronne amincie 51, cela entraîne un retrait du pointeau du plot 62 hors de l'ouverture 23. Les gaz peuvent alors passer par l'ouverture 23 entre la lèvre 27 et le pointeau selon les flèches des Figures 13 et 14. Puis, les gaz sont évacués à l'extérieur en passant par les fenêtres 39 du couvercle 21. Si un filtre est présent dans l'espace intérieur 19 du boîtier 11, les gaz sont alors forcés de passer au travers de ce filtre. Ils trouvent leur chemin jusqu'aux fenêtres 39 grâce aux évidements 49 dans la paroi 41 que le filtre ne peut obstruer en raison de leur forme en retrait.

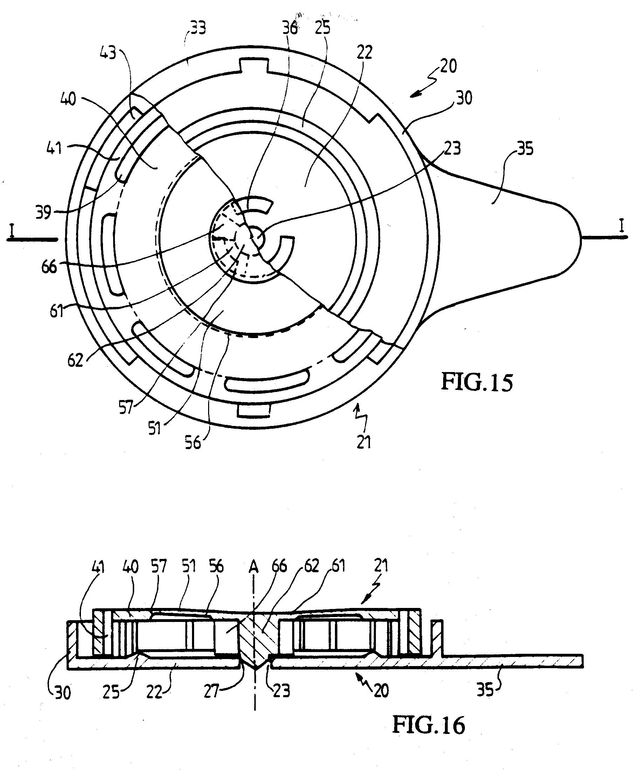

Les Figures 15 et 16 se rapportent quant à elles à la position de fermeture du clapet 50. Dans ce cas, comme le montre bien la vue de dessus de la Figure 15 où, là encore, bossages 66 et rampes 36 sont représentés en pointillés, les bossages 66 s'appuient à l'autre extrémité de la rampe 38 des guides 36, c'est-à-dire sont en position basse. Il s'ensuit que le pointeau du plot 62 peut pénétrer à l'intérieur de l'ouverture 23. Il y est maintenu par la force de rappel assurée par l'effet élastique dû aux conditions de moulage de l'ensemble marge 40 - couronne amincie 51 - disque 61. Par ailleurs, la collaboration du pointeau avec la lèvre 27 assure l'étanchéité de la fermeture. La colonne 34 située à un bout d'un rebord 33 assure de son côté une butée qui empêche de tourner trop le couvercle 51 par rapport au fond 20 et évite que les bossages 66 ne sortent par suite tout à fait des rampes 38.

Dans cette position basse, le clapet 50 peut dans une certaine mesure s'ouvrir automatiquement si la pression des gaz dans la poche devient si forte qu'en s'appliquant à la surface du pointeau au travers de l'ouverture 23, ils parviennent à exercer une force capable de vaincre la force de rappel des moyens élastiques constitués par la couronne amincie 51.

Il est à noter que, dans le premier mode de réalisation du présent dispositif, les gaz s'échappent au travers du filtre lorsque le clapet est fermé et directement par l'ouverture 15 du filtre et sans donc passer au dedans de ce dernier lorsque le clapet est ouvert. Dans le second mode de réalisation, la fermeture du clapet s'oppose à tout échappement de gaz et rend la poche étanche tandis que lorsqu'il est ouvert, les gaz sont obligés de passer à l'intérieur du filtre.

## Revendications

1. Dispositif de dégazage, notamment pour poche de stomie, constitué par un boîtier (11) plat comportant un fond (20) ayant une face interne au boîtier et une face externe au boîtier, adapté à être fixé par ladite face externe sur une face exposée de ladite poche (P) et muni d'une ouverture (23) communiquant avec ladite poche, ainsi qu'un clapet (50) du type fermé au repos, ledit clapet étant formé par un organe de fermeture adapté à s'appliquer sur ladite ouverture (23) dudit fond du côté de ladite face interne et d'un seul tenant avec des moyens élastiques (51, 52) pour repousser ledit organe contre ledit fond (20) avec une force de rappel prédéterminée, caractérisé en ce que le boîtier (11) comporte en outre un couvercle (21) amovible adapté à fermer ledit fond (20) et en ce que le clapet (50) est solidaire dudit couvercle (21).

2. Dispositif selon la Revendication 1, caractérisé en ce que les moyens élastiques (51, 52) consistent en au moins un méplat (51, 52) élastiquement déformable s'étendant entre ledit couvercle (21) et ledit organe de fermeture.

3. Dispositif de dégazage selon la Revendication 2, caractérisé en ce que ledit fond (20) et ledit couvercle (21) sont fabriqués sous forme d'une ébauche d'un seul tenant, de préférence par moulage par injection d'une matière plastique, des ponts de matière reliant dans l'ébauche ledit fond et ledit couvercle de sorte que la distance entre ledit couvercle (21) et ledit organe de fermeture (50) est supérieure à la distance entre ledit fond (20) et ledit couvercle (21) dans le boîtier définitif (11) obtenu en rompant lesdits ponts de matière et en fermant ledit fond avec ledit couvercle.

4. Dispositif de dégazage selon la revendication 2 ou la revendication 3, caractérisé en ce qu'un filtre (12) comportant un trou central (15) est logé dans ledit boîtier (11) de sorte qu'il est pincé entre ledit clapet (50) et ledit fond, son trou venant dans le prolongement de ladite ouverture (23) dudit fond (20).

5. Dispositif de dégazage selon l'une quelconque des Revendications 2 à 4, caractérisé en ce que l'ensemble formé par ledit couvercle (21) et ledit organe de fermeture est conformé pour permettre l'actionnement manuel dudit clapet (50).

6. Dispositif de dégazage selon la Revendication 5, caractérisé en ce que
- ledit boîtier (11) est cylindrique, et en ce que
- ledit couvercle (21) est assujetti audit fond (20) de façon à pouvoir être déplacé relativement audit fond grâce à des moyens d'actionnement manuel selon un mouvement de rotation autour de l'axe (A) du boîtier (11).

7. Dispositif de dégazage selon la Revendication 6, caractérisé en ce que le couvercle (21) est assujetti sur le fond (20) par emboîtement et en ce que les moyens d'actionnement manuel consistent en une oreille (45) en saillie radiale sur le couvercle (21).

8. Dispositif de dégazage selon la Revendication 7, caractérisé en ce que ledit fond (20) comporte une autre oreille (35) en saillie radiale propre à servir de repère d'indexation pour celle du couvercle (20).

9. Dispositif de dégazage selon l'une quelconque des Revendications 6 à 8, caractérisé en ce que
- ledit organe de fermeture (50) est solidaire d'au moins un doigt radial (71) adapté à circuler, lorsque lesdits moyens d'actionnement manuel sont actionnés, dans un guide dudit fond s'étendant circonférentiellement et de forme telle que ledit doigt (71) peut, selon sa position dans ledit guide, être libre ou être soulevé à l'encontre desdits moyens élastiques le long d'une rampe (38) du guide.

10. Dispositif de dégazage selon la Revendication 9, caractérisé en ce qu'il existe aussi une position dans laquelle ledit doigt (71) peut être verrouillé sous un crochet (37) dudit guide.

11. Dispositif de dégazage selon l'une quelconque des Revendications 6 à 8, caractérisé en ce que ledit organe de fermeture consiste en un pointeau, le couvercle (21) comportant également au moins un bossage (66) tandis que ledit fond (20) comporte au moins une rampe hélicoïdale (38) sur laquelle ledit bossage (66) est adapté à circuler entre une position basse et une position haute lorsque le couvercle (21) est tourné par rapport audit fond (20) de sorte que, dans la position basse, ledit pointeau est rappelé par les moyens élastiques pour fermer ladite ouverture (23) tandis que, dans la position haute, ledit pointeau est repoussé hors de l'ouverture (23) à l'encontre desdits moyens élastiques.

12. Dispositif de dégazage selon la Revendication 11, caractérisé en ce qu'il y a trois bossages (66) et trois rampes (38) disposées selon des directions radiales faisant 120° les unes par rapport aux autres.

13. Dispositif de dégazage selon la Revendication 12, caractérisé en ce que lesdits bossages (66) sont solidaires d'un plot (62) qui porte ledit pointeau à son extrémité, les extrémités libres (68) desdits bossages (66) étant en retrait par rapport audit pointeau de façon que ledit pointeau pénètre dans ladite ouverture (23) lorsque lesdits bossages (66) sont en position basse.

14. Dispositif de dégazage selon l'une quelconque des Revendications 11 à 13, caractérisé en ce que ledit boîtier (11) comporte un espace intérieur (19) adapté à recevoir un filtre et limité par une paroi cylindrique (41) comportant des évidements (49) axiaux communiquant avec des fenêtres du dessus du couvercle de sorte que les gaz peuvent s'échapper malgré la présence d'un filtre à l'intérieur du boîtier (11).

15. Dispositif de dégazage selon la Revendication 14, caractérisé en ce que le fond (20) présente un relief (25) intérieur adapté à pincer le filtre de sorte que les gaz sont forcés de passer au travers du filtre pour s'échapper par les évidements (49) et les fenêtres (39).

16. Dispositif de dégazage selon l'une quelconque des Revendications 11 à 15, caractérisé en ce que le couvercle (21) est assujetti sur le fond (20) par un emboîtement à baïonnette.

17. Dispositif de dégazage selon la Revendication 16, caractérisé en ce que ledit fond (20) et ledit couvercle (21) présentent des rebords (33, 43) adaptés à collaborer de sorte qu'une fois ledit fond (20) et ledit couvercle (21) emboîtés, les rebords (33, 43) coopèrent pour les empêcher de se séparer, lesdits rebords (33, 43) s'étendant sur un secteur d'environ 90° et étant disposés de façon diamétralement opposée sur le couvercle (21) et sur le fond (20).

18. Dispositif selon la Revendication 17, caractérisé en ce que le couvercle (21) s'insère à l'intérieur du fond (20), les rebords (43) du couvercle (21) saillant vers l'extérieur tandis que les rebords (33) du fond (20) saillent vers l'intérieur.

19. Dispositif de dégazage selon l'une des Revendications 16 à 18, caractérisé en ce qu'au moins un rebord (33) se prolonge en une colonne axiale (34) de butée adaptée à empêcher que fond (20) et couvercle (21) ne puissent tourner au-delà d'une certaine valeur et que les bossages (66) ne quittent par suite ladite rampe (38).

20. Poche de recueil pour stomie comportant un dispositif (10) de dégazage et de contrôle de la pression interne selon l'une quelconque des Revendications précédentes.

## Claims

1. A degassing device, particularly for a stomal pouch, consisting of a flat casing (11) comprising a bottom (20) having one face inside the casing and one face outside the casing, adapted to be fixed by the said outer face onto an exposed face of the said pouch (P) and provided with an aperture (23) communicating with the said pouch, as well as a valve (50) of the type which is closed when at rest, the valve being formed by a closure member adapted to apply itself to the said aperture (23) in the said bottom on the side of the inner face and constructed in one piece with resilient means (51, 52) for pushing the said member back against the said bottom (20) with a predetermined restoring force, characterised in that the casing (11) comprises furthermore a removable cover (21) adapted to close the said bottom (20) and in that the valve (50) is rigid with the said cover (21).

2. A device according to claim 1, characterised in that the resilient means (51, 52) consist of at least one elastically deformable flattened surface (51, 52) extending between the said cover (21) and the said closure member.

3. A degassing device according to claim 2, characterised in that the said bottom (20) and the said cover (21) are produced in the form of a one-piece blank, preferably by injection moulding of a plastics material, bridges of material connecting the bottom and cover of the blank in such a way that the distance between the said cover (21) and the said closure member (50) is greater than the distance between the said bottom (20) and the said cover (21) in the final casing (11) obtained by breaking the bridges of material and closing the said bottom with the said cover.

4. A degassing device according to claim 2 or claim 3, characterised in that a filter (12) comprising a central hole (15) is so housed in the said casing (11) that it is gripped between the said valve (50) and the said bottom, its hole becoming an extension of the said aperture (23) in the said bottom (20).

5. A degassing device according to any one of claims 2 to 4, characterised in that the assembly formed by the said cover (21) and the said closure member is shaped to allow manual operation of the said valve (50).

6. A degassing device according to claim 5, characterised in that
- the said casing (11) is cylindrical and in that
- the said cover (21) is fastened to the said bottom (20) in such a way that it can be displaced in relation to the said bottom thanks to manual actuating means and according to a rotary movement about the axis (A) of the casing (11).

7. A degassing device according to claim 6, characterised in that the cover (21) is fixed to the bottom (20) by simple interlocking and in that the manual actuating means consist of a radially projecting lug (45) on the cover (21).

8. A degassing device according to claim 7, characterised in that the said bottom (20) comprises another radially projecting lug (35) adapted to serve as an indexing reference for that of the cover (20).

9. A degassing device according to any one of claims 6 to 8, characterised in that
- the said closure member (50) is rigid with at least one radial member (71) adapted to circulate, when the said manual actuating means are operated, in a guide in the said bottom extending circumferentially and of such a form that the said finger (71) can, according to its position in the said guide, be free or be raised against the said resilient means and along a ramp (38) on the guide.

10. A degassing device according to claim 9, characterised in that there is also a position in which the said finger (71) can be locked under a hook (37) on the said guide.

11. A degassing device according to any one of claims 6 to 8, characterised in that the said closure member consists of a needle, the cover (21) likewise comprises at least one boss (66) while the said bottom (20) comprises at least one helical ramp (38) on which the said boss (66) is adapted to move between a low position and a high position when the cover (21) is turned in relation to the said bottom (20) so that, in the low position, the said pointer is restored by elastic means in order to close the said aperture (23) while in the high position the said needle is pushed back out of the aperture (23) against the said resilient means.

12. A degassing device according to claim 11, characterised in that there are three bosses (66) and three ramps (38) disposed in radial positions at 120° in respect of one another.

13. A degassing device according to claim 12, characterised in that the said bosses (66) are integral with a stud (62) which carries the said pointer at its end, the free ends (68) of the said bosses (66) being set back in relation to the pointer so that the said pointer enters the said aperture (23) when the said bosses (66) are in the low position.

14. A degassing device according to any one of claims 11 to 13, characterised in that the said casing (11) comprises a interior space (19) adapted to accommodate a filter and bounded by a cylindrical wall (41) comprising axial cut-outs (49) communicating with windows on the top of the cover so that the gases are able to escape despite the presence of a filter inside the casing (11).

15. A degassing device according to claim 14, characterised in that the bottom (20) comprises an interior relief (25) adapted to pinch the filter so that the gases are forced to pass through the filter in order to escape through the cut-outs (49) and windows (39).

16. A degassing device according to any one of claims 11 to 15, characterised in that the cover (21) is fixed on the bottom (20) by a bayonet type of fitting.

17. A degassing device according to claim 16, characterised in that the said bottom (20) and the said cover (21) comprise returned edges (33, 43) adapted to co-operate so that once the said bottom (20) and the said cover (21) have been fitted together, the edges (33, 43) co-operate to prevent them from becoming separated, the said returned edges (33, 43) extending over a segment of approx. 90° and being disposed in a diametrically opposite relationship on the cover (21) and on the bottom (20).

18. A device according to claim 17, characterised in that the cover (21) fits inside the bottom (20), the returned edges (43) on the cover (21) projecting outwardly while the returned edges (33) on the bottom (20) project inwardly.

19. A degassing device according to one of claims 16 to 18, characterised in that at least one returned edge (33) extends in an axial column (34) which is appropriately in abutment in order to prevent the bottom (20) and the cover (21) being able to turn beyond a certain amount and in that the bosses (66) do not therefore leave the said ramp (38).

20. A stomal collecting pouch comprising a device (10) for degassing and monitoring the internal pressure in accordance with any one of the preceding claims.

## Patentansprüche

1. Entgasungsvorrichtung, insbesondere für Ostomiebeutel, bestehend aus einem flachen Gehäuse (11) mit einem Boden (20), der eine bezüglich des Gehäuses Innenfläche und eine bezüglich des Gehäuses Außenfläche hat, derartiger Auslegung, daß es durch diese Außenfläche gegen eine freiliegende Fläche dieses Beutels (P) fixiert werden kann und mit einer Öffnung (23), die mit diesem Beutel in Verbindung steht, versehen ist sowie eine Ventilklappe (50) vom im Ruhezustand geschlossenen Typ, wobei diese Klappe durch ein Schließorgan geformt ist, das so ausgelegt ist, daß es sich gegen diese Öffnung (23) dieses Bodens auf der Seite dieser Innenfläche legt und in einem Stück mit den elastischen Mitteln (51, 52) ausgebildet ist, um dieses Organ gegen diesen Boden (20) mit einer vorbestimmten Rückstellkraft zurückzustoßen, dadurch gekennzeichnet, daß das Gehäuse (11) im übrigen einen abnehmbaren Deckel (21) umfaßt, derartiger Auslegung, daß er diesen Boden (20) schließt, und daß die Ventilklappe (50) fest mit diesem Deckel (21) verbunden ist.

2. Vorrichtung nach Anspruch 1, dadurch gekennzeichnet, daß diese elastischen Mittel (51, 52) aus wenigstens einer Abflachung (51, 52) bestehen, die elastisch verformbar ist und sich zwischen diesem Deckel (21) und diesem Schließorgan erstreckt.

3. Entgasungsvorrichtung nach Anspruch 2, dadurch gekennzeichnet, daß dieser Boden (20) und dieser Deckel (21) in Form eines Rohlings aus einem Stück, vorzugsweise durch Spritzformen eines elastischen Materials hergestellt sind, wobei Materialbrücken im Rohling diesen Boden und diesen Deckel derart verbinden, daß die Entfernung zwischen diesem Deckel (21) und diesem Schließorgan (50) größer als der Abstand zwischen diesem Boden (20) und diesem Deckel (21) im endgültig erhaltenen Gehäuse (11) wird, indem diese Materialbrücken gebrochen und dieser Boden mit diesem Deckel geschlossen wird.

4. Entgasungsvorrichtung nach Anspruch 2 oder Anspruch 3, dadurch gekennzeichnet, daß ein Filter (12), welches ein mittiges Loch (15) umfaßt, in diesem Gehäuse (11) derart gelagert ist, daß es zwischen dieser Ventilklappe (50) und diesem Boden eingeklemmt ist, wobei sein Loch in die Verlängerung dieser Öffnung (23) dieses Bodens (20) gelangt.

5. Entgasungsvorrichtung nach einem der Ansprüche 2 bis 4, dadurch gekennzeichnet, daß die durch diesen Deckel (21) und dieses Schließorgan gebildete Anordnung so gestaltet ist, daß die Handbetätigung dieser Ventilklappe (50) möglich wird.

6. Entgasungsvorrichtung nach Anspruch 5, dadurch gekennzeichnet, daß dieses Gehäuse (11) zylindrisch ist und daß dieser Deckel (21) an diesem Boden (20) derart befestigt ist, daß der relativ zu diesem Boden dank Handbetätigungsmitteln gemäß einer Drehbewegung um die Achse (A) des Gehäuses (11) beweglich ist.

7. Entgasungsvorrichtung nach Anspruch 6, dadurch gekennzeichnet, daß der Deckel (21) am Boden (20) durch Einstecken befestigt ist und daß diese Handbetätigungsmittel aus einer Lasche (45) bestehen, die gegen den Deckel (21) radial vorsteht.

8. Entgasungsvorrichtung nach Anspruch 7, dadurch gekennzeichnet, daß dieser Boden (20) im übrigen eine Lasche (35) umfaßt, die radial vorsteht und so ausgebildet ist, daß sie als Einstellmarke für die des Deckels (20) dient.

9. Entgasungsvorrichtung nach einem der Ansprüche 6 bis 8, dadurch gekennzeichnet, daß dieses Schließorgan (50) mit wenigstens einem radialen Finger (71) fest verbunden ist, der so ausgebildet ist, daß er, wenn die Handbetätigungsmittel betätigt werden, in einer sich in Umfangsrichtung des Bodens erstreckenden Führung zirkuliert und von einer Form derart ist, daß dieser Finger (71) entsprechend der Position in dieser Führung frei sein kann oder entgegen diesen elastischen Mitteln längs einer Rampe (38) der Führung angehoben werden kann.

10. Entgasungsvorrichtung nach Anspruch 9, dadurch gekennzeichnet, daß auch eine Position existiert, in der dieser Finger (71) unter einem Haken (37) dieser Führung verriegelt werden kann.

11. Entgasungsvorrichtung nach einem der Ansprüche 6 bis 8, dadurch gekennzeichnet, daß dieses Schließorgan aus einer Nadel oder einem Stift besteht, wobei der Deckel (21) auch wenigstens einen Höcker (66) umfaßt, während dieser Boden (20) wenigstens eine Spiralrampe (38) umfaßt, auf der dieser Höcker (66) gemäß seiner Ausgestaltung zwischen einer niedrigen Position und einer hohen zirkulieren oder sich bewegen kann, wenn der Deckel (21) bezüglich des Bodens (20) in die untere Stellung gedreht wird, wobei der Höcker durch die elastischen Mittel rückgestellt wird, um diese Öffnung (23) zu schließen, während in der oberen Stellung diese Nadel bzw. dieser Stift aus der Öffnung (23) entgegen diesen elastischen Mitteln zurückgedrückt wird.

12. Entgasungsvorrichtung nach Anspruch 11, dadurch gekennzeichnet, daß drei Höcker (66) und drei Rampen (38) vorgesehen sind, die entsprechend radialen Richtungen, die einen Winkel von 120° bezüglich einander haben, angeordnet sind.

13. Entgasungsvorrichtung nach Anspruch 12, dadurch gekennzeichnet, daß diese Höcker (66) fest bezüglich eines Klotzes (62) sind, der diese Nadel bzw. diesen Stift an seinem Ende trägt, wobei die freien Enden (68) dieser Höcker (66) bezüglich dieses Stiftes derart zurückspringen, daß dieser Stift in diese Öffnung (23) eindringt, wenn die Höcker (66) sich in unterer Stellung befinden.

14. Entgasungsvorrichtung nach einem der Ansprüche 11 bis 13, dadurch gekennzeichnet, daß dieses Gehäuse (21) einen Innenraum (19) umfaßt, der so ausgelegt ist, daß er ein Filter aufnimmt und durch eine zylindrische Wand (41) begrenzt ist, welche axiale Ausnehmungen (49) umfaßt, die mit Fenstern auf der Oberseite des Deckels derart in Verbindung stehen, daß die Gase trotz des Vorhandenseins eines Filters im Inneren des Gehäuses (11) entweichen können.

15. Entgasungsvorrichtung nach Anspruch 14, dadurch gekennzeichnet, daß der Boden (20) ein inneres Reliefs (25) aufweist, das so ausgebildet ist, daß es das Filter derart klemmt, daß die Gase veranlaßt werden, durch das Filter zu gehen, um über Ausnehmungen (49) und die Fenster (39) zu entweichen.

16. Entgasungsvorrichtung nach einem der Ansprüche 11 bis 15, dadurch gekennzeichnet, daß der Deckel (21) am Boden (20) über einen Bajonettverschluß befestigt ist.

17. Entgasungsvorrichtung nach Anspruch 16, dadurch gekennzeichnet, daß dieser Boden (20) und dieser Deckel (21) Ränder (33, 43) einer Auslegung derart aufweisen, daß sie so zusammenwirken, daß, wenn einmal dieser Boden (20) und dieser Deckel (21) ineinander eingesteckt sind die Ränder (33, 43) zusammenwirken, um sie daran zu hindern, sich voneinander zu trennen, wobei diese Ränder (33, 43) sich über einen Sektor von etwa 90° erstrecken und einander diametral gegenüber auf dem Deckel (21) und auf dem Boden (20) angeordnet sind.

18. Entgasungsvorrichtung nach Anspruch 17, dadurch gekennzeichnet, daß der Deckel (21) sich in das Innere des Bodens (20) einfügt, die Ränder (43) des Deckels (21) nach außen vorstehen, während die Ränder (33) des Bodens (20) nach innen vorstehen.

19. Entgasungsvorrichtung nach einem der Ansprüche 16 bis 18, dadurch gekennzeichnet, daß wenigstens ein Rand (33) in einer axialen Anschlagsäule (34) sich verlängert, einer Auslegung derart, daß der Boden (20) und Deckel (21) sich nicht über einen gewissen Wert hinaus drehen können und daß die Höcker (66) somit diese Rampe (38) nicht verlassen.

20. Sammelbeutel für Ostomie, eine Vorrichtung (10) zur Entgasung und zur Regelung des Innendrucks gemäß einem der vorhergehenden Ansprüche umfassend.
